# EUROPEAN PATENT APPLICATION

(11) **EP 4 190 320 A1**
(43) Date of publication of application: **07.06.2023**
(21) Application number: 20947537.5
(22) Date of filing: 27.07.2020
(51) Int. Cl.: A61K 31/137, A61P 3/04, A61P 25/26

(54) **CRYSTALLINE FORM OF PHENTERMINE HYDROCHLORIDE AND PROCESS FOR OBTAINING SAME**

(71) Applicant: Eurofarma Laboratórios S.A., 06696-000 Itapevi - SP (BR)
(72) Inventor: DE OLIVEIRA FARIA, Luiz Felipe, CEP 05468-909 São Paulo (BR); GERÔNIMO KOBATA, Pedro Yuri, CEP 18046-485 Sorocaba (BR)
(74) Representative: KATZAROV S.A.
(86) International application number: PCT/BR2020/050290
(87) International publication number: WO 2022/020910

(57) **Abstract**

The present invention relates to the crystalline form of phentermine hydrochloride monohydrate and processes for obtaining said crystalline form. Phentermine or 2-methyl-1-phenylpropan-2-amine is found in the hydrochloride salt form, being a central nervous system stimulant, used as an appetite suppressant and stimulant in medicaments. The present invention belongs to the fields of chemistry and pharmacy.

## Description

### Field of the invention

The present invention relates to the crystalline form of phentermine hydrochloride monohydrate and the process for obtaining said crystalline form. The present invention is in the fields of chemistry and pharmacy.

### Background of the invention

Phentermine or 2-methyl-1-phenylpropan-2-amine is commonly found in the hydrochloride salt form (#CAS 1197-21-3) and it is a central nervous system stimulant used as an appetite suppressant and stimulant in medicaments. Such a compound and its synthetic route are described in the U.S. Pat No. 2,408,345, whose priority date is April 13, 1942. It has the molecular form C10H16CIN and the following structure:

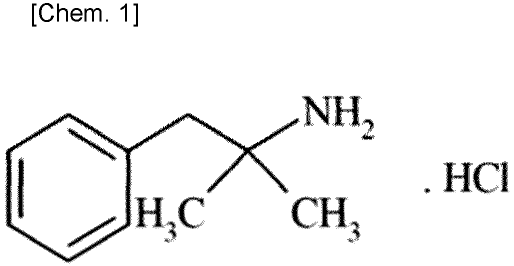

Polymorphic forms of a given substance may have different chemical and physical properties, such as melting point, chemical reactivity, solubility, dissolution rate, rheological properties and density. These properties can directly affect the processability and manufacture of the active and the pharmaceutical form containing said active, as well as stability, dissolution and bioavailability. In this way, polymorphism can impact the quality, safety and efficacy of a pharmaceutical product.

The term polymorphism, used in the field of pharmaceutical and chemical sciences, is defined as the ability of chemical compounds to exist in two or more crystalline structures. When the solvent is incorporated into the compound's crystalline network in stoichiometric or non-stoichiometric amounts, molecular adducts called solvates, also called pseudopolymorphs, are formed. Solvates with incorporated water molecules, on the other hand, are called hydrates. According to the number of incorporated molecules, the hydrate has a different name, such as: monohydrate - 1 molecule of water; sesquihydrate - 1.5 molecules of water, dihydrate - 2 molecules of water, and so on.

Several methodologies can be used to characterize an active pharmaceutical ingredient polymorphs'. Demonstration of a distinct structure by single crystal X-ray diffraction is considered definitive evidence for polymorphism. However, polycrystal X-ray diffraction in conjunction with other techniques including microscopy, thermal analysis (e.g., differential scanning calorimetry, thermogravimetric analysis, and hot stage microscopy), and spectroscopy (e.g., infrared absorption, Raman scattering, and nuclear magnetic resonance) can also be used for crystalline forms' characterization.

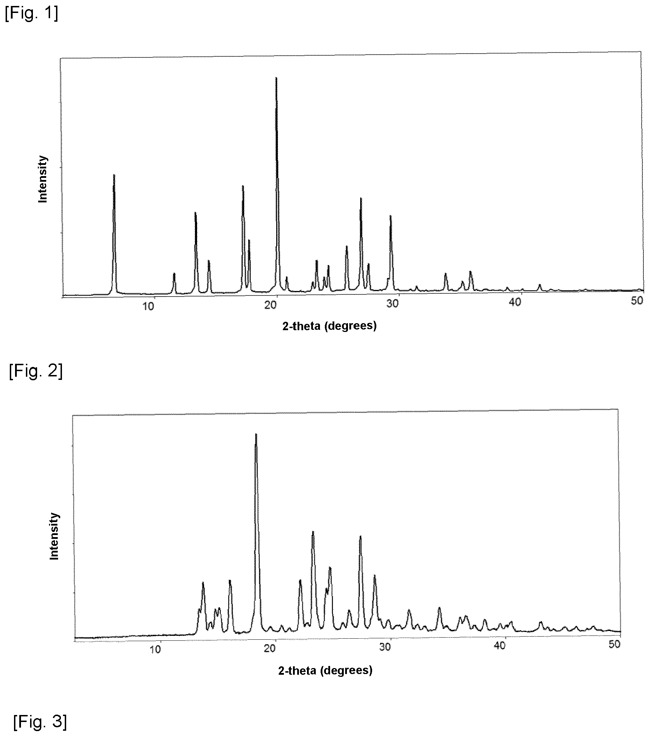

An anhydrous crystal form of phentermine hydrochloride, characterized by the X-ray diffraction shown in Figure 1, is the only crystal form of phentermine hydrochloride described in the state of the art. In an European Agency's document that refers to a formulation comprising phentermine and topiramate (process No EMEA/H/C/002350/0000), it is mentioned that there is no evidence of polymorphism associated with phentermine hydrochloride, which makes the present invention surprising.

U.S. Pat. Application 2008/293695 describes many phentermine salts and their polymorphs such as phentermine pamoate, phentermine xinafoate, phentermine salicylate. Additionally, U.S. Pat. No 8,334,322 specifically describes phentermine pamoate polymorph. Nonetheless, none of these documents disclose polymorphs or pseudopolymorphs of phentermine hydrochloride.

Phentermine hydrochloride in anhydrous form has a gain of about 7.5% by mass at 80% relative humidity, being classified as hygroscopic according to the British Pharmacopoeia, 2016 edition (British Pharmacopoeia). This fact can cause dosage problems if the active is manipulated under high humidity conditions. These handling and hygroscopicity problems bring to light the need for new solid forms of phentermine hydrochloride that provides better processing properties, such as ease of handling, storage stability and ease of purification.

Crystal forms' diversity of a given active increases the portfolio of materials available to the formulation scientist optimize pharmaceutical formulations, since there are differences among crystal forms. As an example, one can cite polymorph's different crystalline habits, greater crystallinity, less susceptibility to humidity and stability, technical features responsible for improving the manufacturing process and manipulation, dissolution profile and shelf-life.

### Brief description of figures

[Fig. 1] shows the X-ray diffractogram of the anhydrous crystalline form of phentermine hydrochloride.
[Fig. 2] shows the characteristic X-ray diffractogram of the crystalline form of phentermine hydrochloride monohydrate.
[Fig. 3] shows the comparison between the X-ray diffractograms of the anhydrous form (A) and the monohydrate form (B) of phentermine hydrochloride.
[Fig. 4] shows the characteristic infrared absorption spectra of the anhydrous (A) and monohydrate (B) forms of phentermine hydrochloride.
[Fig. 5] shows the characteristic Raman spectrum of the phentermine hydrochloride monohydrate form.
[Fig. 6] shows the crystalline habits of phentermine hydrochloride for the anhydrous form (A) and the monohydrated form obtained by the processes of suspension in water under room conditions (B) and recrystallization in water at low temperature (°C).
[Fig. 7] shows the thermogravimetric analysis (TGA) curves obtained with a heating rate of 10 °C/min for phentermine hydrochloride in both anhydrous form (dotted line) and monohydrate form (solid line) of phentermine hydrochloride.
[Fig. 8] shows the differential scanning calorimetry (DSC) curves obtained with a heating rate of 10 °C/min for phentermine hydrochloride in both anhydrous form (dotted line) and monohydrate form (solid line) of phentermine hydrochloride.
[Fig. 9] shows the dynamic water vapor desorption curve at relative humidity from 95% to 0% in steps of 5% at 25 °C for the phentermine hydrochloride monohydrate form. In all humidity conditions, the equilibrium criterion of mass variation of less than 0.001% was reached.
[Fig. 10] shows X-ray diffractograms of phentermine hydrochloride in anhydrous form (A) and monohydrate form after heating at 60 °C (B), when it converts to anhydrous form.
[Fig. 11] shows the photographs of hot stage optical microscopy obtained during heating at 10 °C/min of the phentermine hydrochloride monohydrate form. As shown in the highlights, during the transition from monohydrate form (15 °C) to anhydrous form (66 °C), a change in crystal's appearance is observed, however, without changes in the crystalline habit.
[Fig. 12] shows the infrared absorption spectra for phentermine hydrochloride monohydrate form before incubation (A) and after incubation in an oven at 50 °C and 75% relative humidity for 30 days (B).
[Fig. 13] shows dynamic water vapor adsorption curves at relative humidity of 60%, 80% and 95% at 25 °C for phentermine hydrochloride salts in anhydrous form (dotted line) and monohydrate form (solid line). In all humidity conditions, the equilibrium criterion of mass variation of less than 0.001% was reached.

### Summary of the invention

The present disclosure provides a crystalline form of phentermine hydrochloride monohydrate and processes for obtaining the same. The said crystalline form promotes advantages to phentermine hydrochloride due to its stability and lower hygroscopicity when compared to the anhydrous form of the same salt, the monohydrated form can also be produced in different crystalline habits (prismatic or placoid) depending on the method of production used, promoting better processing properties such as ease of handling and storage stability. Furthermore, the active's bioavailability in several pharmaceutical compositions, such as tablets, gelatin capsules, powders, granules, solutions or suspensions can also be improved.

The present invention provides the following objects as inventive concepts.

A first object of the invention is the crystalline form of phentermine hydrochloride in the monohydrated form. A second object of the invention are processes for obtaining the crystalline form of phentermine hydrochloride as described in the first object and in its embodiments.

### Detailed description of the invention

The present invention consists of the definition of a new crystalline form of phentermine hydrochloride monohydrate, obtained in an unexpected and surprising way, since phentermine's molecule was first described in 1942 and no further crystalline forms of its hydrochloride salt have been described in the state of the art. Additionally, processes for obtaining said crystalline form are described, preferably high shear granulation and drying in a fluidized bed.

The crystalline form of phentermine hydrochloride monohydrate is obtained from the anhydrous salt form by recrystallization of suspension or solution thereof in pure water or solvent mixture containing water, or by wet granulation process using high shear. The remaining crystalline material is dried in an oven or fluidized bed to remove solvent or residual water. Alternatively, it is possible to use a mixture of water with more volatile solvents in the obtaining process to reduce the time of the drying process.

Another way of obtaining the crystalline form of the present invention is exposing the anhydrous form to an environment with a high humidity content (above 90% RH) using a chamber with controlled humidity.

Further widely used methods in the pharmaceutical industry, such as lyophilization, drying via spray drying and drying in a vacuum oven, are viable alternatives for preparing the crystalline form of the present invention.

In the present invention, the term "monohydrate" refers to the hydrate in which a water molecule is incorporated into the crystalline structure, associated with a phentermine hydrochloride molecule, wherein the stoichiometry is 1:1.

In the present invention, the expression "peaks expressed in degrees 2-theta" refers to the characteristic peaks of the X-ray diffractogram obtained using a wavelength of 1.54 Å (Cu K-alpha) of the phentermine hydrochloride monohydrate form.

In a first object, the crystalline form of phentermine hydrochloride is the monohydrate form. In one embodiment, the crystalline form comprises peaks expressed in degrees 2-theta (± 0.2°) at 13.3; 13.7; 14.3; 14.7; 15.1; 16.0; 18.4; 19.5; 20.5; 21.2; 22.2. 22.7; 23.3; 24.4; 24.8; 25.8; 26.4; 27.4; 28.2; 28.6; 29.1; 29.8; 30.5; 31.6; 32.3; 32.9; 34.2; 34.9; 36.0; 36.6; 37.3; 38.2; 39.5; 40.0; 40.4; 43.1; 43.6; 45.1; 46.1; 47.1; 47.6; 48.9; 52.9 and 54.2 on its X-ray diffractogram. In one embodiment, the crystalline form comprises specific peaks expressed in degrees 2-theta (± 0.2°) at 13.7; 14.7; 15.1; 16.0; 18.4; 22.2; 24.8; 26.4; 28.6 and 29.8.

In one embodiment, the crystalline form comprises the bands 849, 918, 1030, 1074, 1082, 1151, 1173, 1181, 1134, 1380, 1396, 1449, 1454, 1468, 1528, 1533, 1618, 1632, 1650, 2804, 2894, 2935, 2973, 2987, 3066, 3144, 3148 and 3415 cm-1 in their absorption spectrum in the infrared region wherein the band at 3415 cm-1 refers to the water molecule.

In a second object, the process of obtaining the crystalline form of phentermine hydrochloride defined in the first object and in its embodiments comprises the steps of: (a) High shear granulation of phentermine hydrochloride in anhydrous form; (b) Addition of granulating solution in the mixture of the granulate from (a); (c) Mixture of the granulate of (a) and the granulating solution of (b) to obtain a powder; and, (d) Drying of the powder in a fluidized bed or in an oven or in a vacuum oven.

In one embodiment, said granulating solution of step (b) is water added at a rate equal to or above 5 mL/min and step (c) takes place at a mixing speed of at least 100 rpm. In a preferred embodiment, the mixing speed ranges from 100 to 150 rpm, more specifically 150 rpm. In another embodiment, the water addition rate ranges from 5 to 30 mL/min. In one embodiment, in step (d), the drying of the powder is in a fluidized bed with a maximum inlet air temperature of 60 °C and the drying of the material occurs until the obtention of a pure monohydrated form. In a preferred embodiment, drying occurs between 5 and 15 min. In a preferred embodiment, the process parameters can be jointly optimized to obtain the pure monohydrate form.

Alternatively, the process for obtaining the crystalline form of phentermine hydrochloride comprises the steps of: (a) Recrystallization of the anhydrous form suspension or solution of the of phentermine hydrochloride in water or in a mixture of water and volatile solvents; and (b) Drying in an oven or vacuum oven or vacuum filtration of the solution.

In one embodiment, the said recrystallization of the solution occurs at low temperature or with heating and the said recrystallization of the suspension is maintained under magnetic stirring, at a temperature of 25 °C for 24h. In another embodiment, said low temperature recrystallization takes place between 2 and 8 °C. In another embodiment, said recrystallization is performed with heating above 60 °C.

In one embodiment, said volatile solvents of step (a) are selected, without limitation, from the group consisting of methanol, ethanol, propanol, isopropanol, acetone, dichloromethane or a mixture thereof.

Alternatively, the obtaining process may comprise the steps of: (a) Exposure of the anhydrous form of phentermine hydrochloride to an atmosphere with high humidity conditions in a controlled humidity chamber; and (b) Drying in an oven or vacuum oven.

In one embodiment, said high humidity condition ranges from 90 to 95% RH. In a preferred embodiment, said humidity condition is 95% RH.

The crystalline form of phentermine hydrochloride monohydrate was characterized and differentiated from the anhydrous form using X-ray diffraction techniques, infrared absorption spectrometry, Raman spectroscopy, thermal analysis (thermogravimetry and differential scanning calorimetry), accelerated stability study, optical microscopy and hot stage optical microscopy and dynamic water vapor sorption experiments.

Table 1 contains the angles and relative intensity of the main peaks in the X-ray diffractogram (Figure 2) that characterize the crystalline form of phentermine hydrochloride monohydrate.

**[Table 1] Characteristic Bragg diffraction peaks of the phentermine hydrochloride monohydrate form.**

| **2 - theta (*λ* = 1.54 Å) (degrees)** | **Distance (Angstroms)** | **Relative intensity (arbitrary units)** |
|---|---|---|
| 13.3 | 6.63 | 10.08 |
| 13.7 | 6.45 | 25.85 |
| 14.3 | 6.19 | 5.64 |
| 14.7 | 6.00 | 12.48 |
| 15.1 | 5.85 | 9.97 |
| 16.0 | 5.52 | 25.54 |
| 18.4 | 4.81 | 100.00 |
| 19.5 | 4.55 | 2.25 |
| 20.5 | 4.33 | 1.78 |
| 21.2 | 4.19 | 0.78 |
| 22.2 | 4.01 | 24.13 |
| 22.7 | 3.92 | 3.60 |
| 23.3 | 3.81 | 50.97 |
| 24.4 | 3.65 | 12.64 |
| 24.8 | 3.59 | 31.75 |
| 25.8 | 3.45 | 1.72 |
| 26.4 | 3.37 | 8.46 |
| 27.4 | 3.25 | 40.78 |
| 28.2 | 3.16 | 2.61 |
| 28.6 | 3.11 | 24.02 |
| 29.1 | 3.07 | 3.03 |
| 29.8 | 3.00 | 3.55 |
| 30.5 | 2.92 | 2.04 |
| 31.6 | 2.82 | 9.92 |
| 32.3 | 2.77 | 2.35 |
| 32.9 | 2.72 | 1.70 |
| 34.2 | 2.62 | 11.12 |
| 34.9 | 2.57 | 2.30 |
| 36.0 | 2.50 | 4.96 |
| 36.6 | 2.45 | 10.34 |
| 37.3 | 2.41 | 2.09 |
| 38.2 | 2.35 | 4.80 |
| 39.5 | 2.28 | 3.24 |
| 40.0 | 2.25 | 1.78 |
| 40.4 | 2.23 | 6.06 |
| 43.1 | 2.10 | 5.69 |
| 43.6 | 2.07 | 1.41 |
| 45.1 | 2.01 | 1.93 |
| 46.1 | 1.97 | 1.88 |
| 47.1 | 1.93 | 0.78 |
| 47.6 | 1.91 | 2.66 |
| 48.9 | 1.86 | 0.47 |
| 52.9 | 1.73 | 1.49 |
| 54.2 | 1.69 | 0.63 |

Figure 3 shows the X-ray diffractograms for the anhydrous and monohydrate forms of phentermine hydrochloride are shown together, illustrating the difference in the position of the diffraction peaks for the different crystalline forms, with the main peaks that characterize the anhydrous crystalline form expressed in degrees 2-theta they are (± 0.2°) 6.8; 11.6; 14.5; 17.3; 17.7; 20.1; 26.9; 29.4; 33.8 and 35.2 and the main peaks that characterize the monohydrated form are 13.7; 14.7; 15.1; 16.0; 18.4; 22.2; 24.8; 26.4; 28.6 and 29.8.

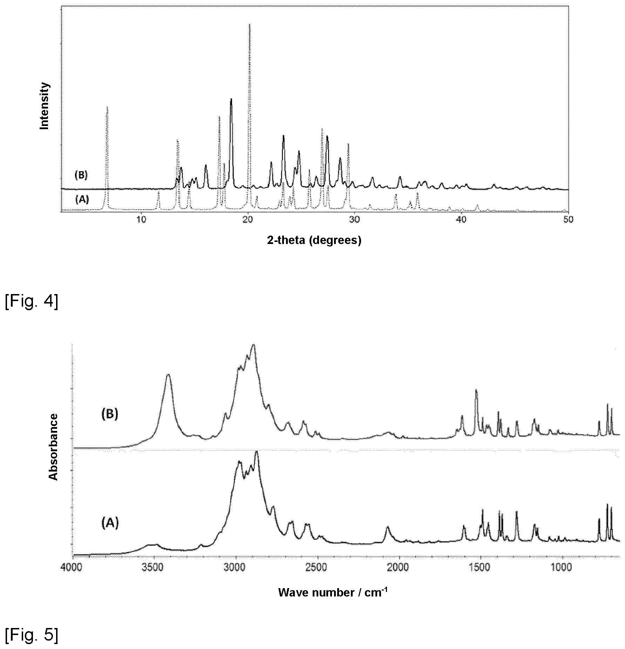

The absorption spectrum in the infrared region of the phentermine hydrochloride monohydrate form is shown in Figure 4 along with the spectrum for the anhydrous salt form. The main bands in the spectrum that characterize the compound in the monohydrate form are: 849, 918, 1030, 1074, 1082, 1151, 1173, 1181, 1134, 1380, 1396, 1449, 1454, 1468, 1528, 1533, 1618, 1632, 1650, 2804, 2894, 2935, 2973, 2987, 3066, 3144, 3148 and 3415 cm-1. Particularly, the intense band at 3415 cm-1 is characteristic of water molecules present in the crystal lattice of the monohydrate form. Residual moisture water molecules are evidenced by a less intense larger band above 3500 cm-1 in the spectrum for the anhydrous form, which appears only as a shoulder of the intense band at ~3415 cm-1 in the spectrum for the monohydrate form.

The Raman spectrum of the crystalline form of phentermine hydrochloride in the monohydrate form is shown in Figure 5 with some of the bands in the Raman spectrum that characterize the compound in the monohydrate form being: 263, 286, 329, 396, 451, 512, 608, 828, 890, 849, 949, 1031, 1073, 1153, 1169, 1335, 1359, 1396, 1444, 1469, 1531, 1603, 2862, 2936, 2988 and 3027 cm-1, mainly the band at 1153 cm-1.

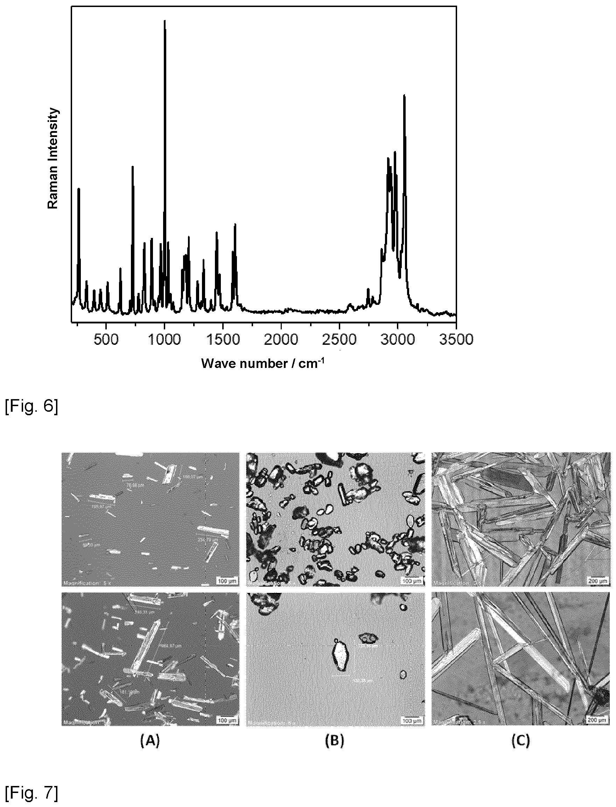

The analysis of the crystalline habit was carried out by optical microscopy, aiming to define the crystalline habit of the monohydrated form and compare it with that of the anhydrous form. In this analysis, it was proved that different habits could be obtained for the monohydrated form depending on the process of obtaining it. Figure 6 shows micrographs for the crystalline habits of the anhydrous and monohydrated forms, with the anhydrous form (A) having a stick habit (prismatic) and the monohydrated form having a placoid or prismatic habit when obtained by suspension under room conditions or (B) by the process of recrystallization in water at low temperature (C), respectively.

The thermal analysis performed through thermogravimetry (Thermogravimetric Analysis, TGA) (Figure 7) indicates that the crystalline form of the present invention contains one water molecule per molecule of the active ingredient. Considering 100 mg of sample, the loss by mass of 8.96% at 120 °C relative to the loss of water corresponds to 8.96 mg, that is, 0.000497 mols of water (whose molar mass is 18.01 g /mol). The remaining mass, that is, 91.04 mg, is attributed to phentermine hydrochloride, whose molar mass is 185.69 g/mol, corresponding to 0.000490 mol of active. Thus, the ratio between phentermine hydrochloride and water (0.000497 mol:0.000490 mol) proves the stoichiometry of 1:1, that is, the existence of phentermine hydrochloride monohydrate. Also in Figure 7, it is observed that the anhydrous form presents a loss of only 1.3% by mass at 120 °C attributed to the loss of residual moisture water molecules.

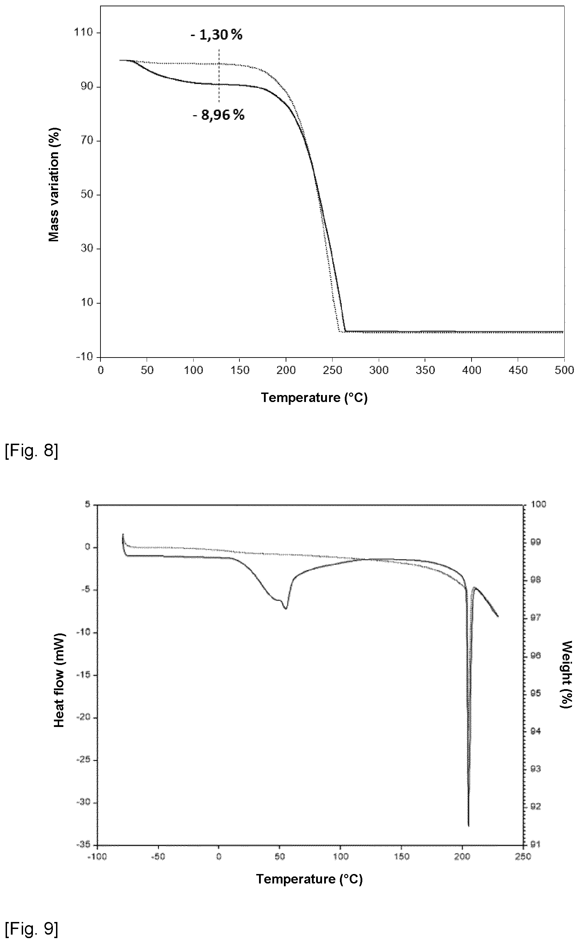

The Differential Scanning Calorimetry (DSC) experiment (Figure 8) is performed under an inert atmosphere using nitrogen flow. Therefore, for phentermine hydrochloride monohydrate, such flow favors water loss, and the experiment will have underestimated results. The experiment would be optimally carried out using an atmosphere with 50% relative humidity (RH), to guarantee the integrity of the monohydrated form, as can be seen in the dynamic sorption experiments of water vapor, the monohydrated form is stable in relative humidity higher than 50% (Figure 9).

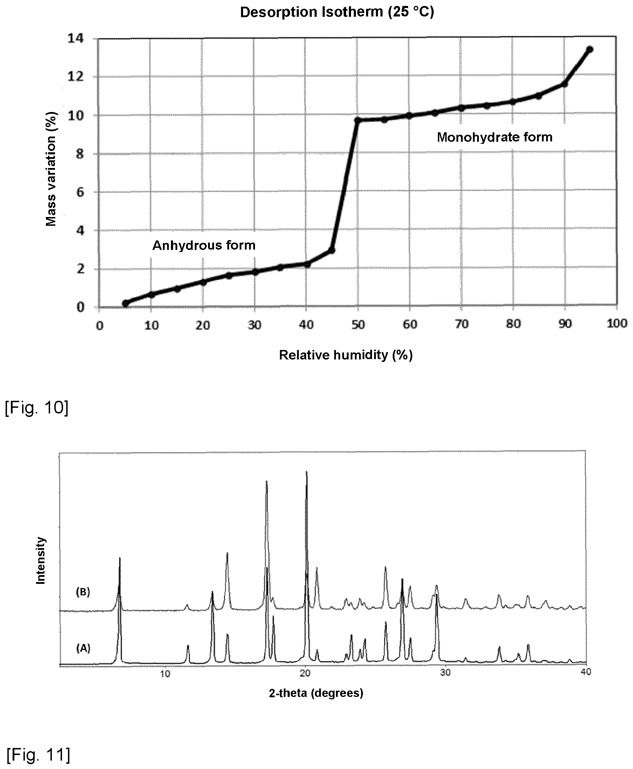

The DSC curve showed that, in the heating process, the monohydrate form converts to the anhydrous form above 50 °C, as indicated by the endothermic event in the DSC curve and by X-ray diffraction measurements performed with the sample of the monohydrate form submitted to heating at 60 °C with subsequent cooling to room temperature (Figure 10). Furthermore, the melting of the anhydrous form occurs around 200 °C in which the active also starts the decomposition process.

Such conversion from the monohydrate form to anhydrous form can also be monitored via hot stage optical microscopy, as shown in Figure 11, which shows that the crystal becomes opaquer around 50 °C, however, without changes in the crystalline habit. Furthermore, melting starts at 200 °C, the same temperature observed in the DSC experiment, and is completed at around 210 °C at the heating rate used in the experiment (10 °C/min), proving the conversion.

Therefore, from the DSC experiments and hot stage microscopy results, the conversion from the monohydrate form to the anhydrous form can be observed.

### EXAMPLES:

The following examples serve to illustrate aspects of the present invention without having, however, any limiting character.

### Example 1 - Process for obtaining the monohydrated crystalline form of phentermine hydrochloride using "high shear" and drying in a fluidized bed.

100 g of phentermine hydrochloride in anhydrous form was granulated using a high shear granulation process. Initially, the homogenization of the raw material was carried out for 3 minutes, under conditions of mixing speed of 150 rpm and chopper speed of 1500 rpm, maintained throughout the process. Next, the granulating solution (20 mL of deionized water) was added for a period of 4 minutes (5 mL/min). The granulated active powder was transferred to a fluidized bed, where it remained for 10 min, with an inlet air temperature of 35 °C and an inlet air flow of 20%, having time to clean the filters with beat every 15 seconds, keeping the powder temperature close to 25 °C. The drying time must be adjusted according to the granulate final humidity which guarantees the obtention of the pure monohydrated form. The best drying time was defined by sample aliquots taken throughout the process. Times of 5, 10 and 15 minutes were checked and it was found that 10 minutes is the preferred time for such conditions. The granulate can be sampled and moisture checked using a moisture analyzer or a drying oven. The final product was characterized by infrared absorption spectroscopy and X-ray diffraction consistently indicating the formation of the phentermine hydrochloride monohydrate form with a placoid habit.

The process parameters can be jointly optimized to obtain the pure monohydrate form.

### EXAMPLE 2 - Process for obtaining the monohydrate crystalline form of phentermine hydrochloride in aqueous suspension.

25 g of phentermine hydrochloride in anhydrous form were added to 50 mL of deionized water at a temperature of 25 °C. The obtained suspension was maintained under magnetic stirring at room conditions (25 °C) for 24 hours. Microscopic analysis indicated a change in crystalline habit to a placoid shape. The suspension was vacuum filtered and the recovered crystals were characterized by infrared absorption spectroscopy and X-ray diffraction consistently indicating the formation of the phentermine hydrochloride monohydrate form with a placoid habit.

### EXAMPLE 3 - Process for obtaining the monohydrated crystalline form of phentermine hydrochloride by recrystallization in aqueous solution at low temperature.

25 g of phentermine hydrochloride in anhydrous form was dissolved in 100 mL of deionized water at a temperature of 25 °C. The solution was maintained cooled in an ice bath at a temperature of 2 to 8 °C for a period of 24 hours. The formation of long prismatic crystals was observed at the bottom of the solution container. The solution was vacuum filtered and the recovered crystals were characterized by infrared absorption spectroscopy and X-ray diffraction consistently indicating the formation of the phentermine hydrochloride monohydrate form with prismatic habit.

### EXAMPLE 4 - Process for obtaining the monohydrate crystalline form of phentermine hydrochloride by recrystallization from an aqueous solution with heating.

25 g of phentermine hydrochloride in anhydrous form was dissolved in 100 mL of deionized water at a temperature of 25 °C. The solution was maintained on a heating plate at a temperature of 60 °C for a period of 24 hours. Crystal formation was observed at the bottom of the solution container after a few hours. The solution was then vacuum filtered and the recovered crystals were characterized by infrared absorption spectroscopy and X-ray diffraction consistently indicating the formation of the phentermine hydrochloride monohydrate form with prismatic habit.

### EXAMPLE 5 - Process for obtaining the monohydrated crystalline form of phentermine hydrochloride in a chamber with controlled relative humidity.

100 mg of phentermine hydrochloride in anhydrous form was maintained in a chamber with a controlled relative humidity of 95% and a temperature of 25 °C for a period of 24 hours. The final product was characterized by infrared absorption spectroscopy and X-ray diffraction consistently indicating the formation of the phentermine hydrochloride monohydrate form with a prismatic habit.

### EXAMPLE 6 - Studies of accelerated stability.

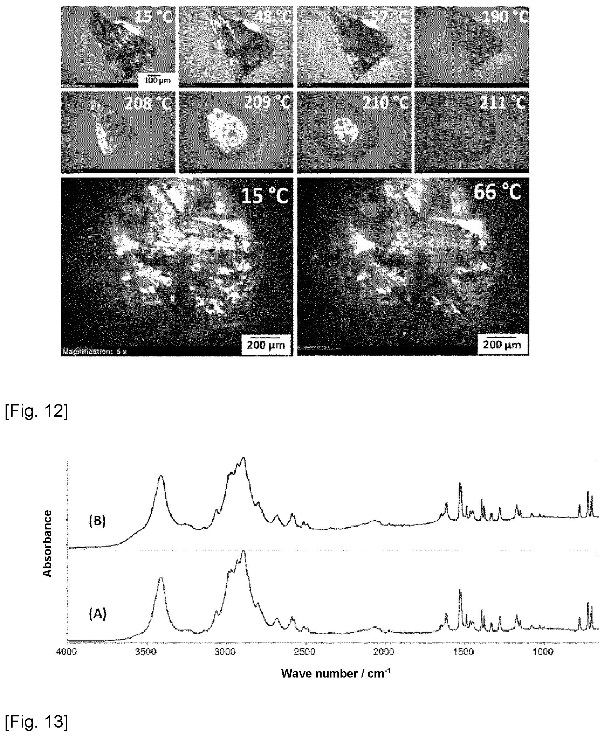

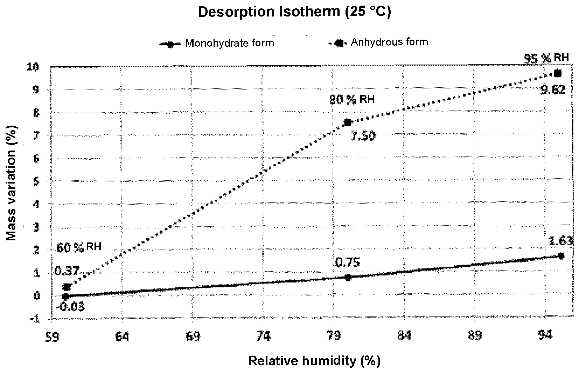

Phentermine hydrochloride monohydrate's stability was tested by keeping 25 g of sample in an oven under conditions of 50 °C and 75% relative humidity for 30 days, as recommended by the FDA guide (Food and Drug Administration, Guidance for Industry Q1A(R2) Stability Testing of New Drug Substances and Products, November 2003 rev.2). After the accelerated stability test was carried out, it was observed by absorption spectroscopy in the infrared region (Figure 12) and X-ray diffraction that there was no polymorphic conversion to anhydrous form or any other polymorphic form of the salt. In addition, DVS (Dynamic Vapor Sorption) experiments proved that both forms are stable.

### EXAMPLE 7 - Stability study of the monohydrated form as a function of the relative humidity (experiment of dynamic adsorption of water vapor).

Figure 13 presents dynamic water vapor sorption experiments under different humidity conditions for the anhydrous and monohydrate forms of phentermine hydrochloride. It is noted that in all analyzed humidity conditions, the system reached equilibrium, with the monohydrate form being much less hygroscopic than the anhydrous form. Phentermine hydrochloride in anhydrous form has a gain of about 7.5% by mass at 80% relative humidity, being classified as hygroscopic according to the British Pharmacopoeia (BF) (British Pharmacopoeia, Ed. 2016), which can cause dosing problems if the active is manipulated in high humidity conditions. In addition, the fact that the anhydrous form is hygroscopic allows its conversion to the monohydrate form in case of exposure to excessive amounts of water. On the other hand, the monohydrate form of the present invention has a gain of about 0.75% by mass when exposed to 80% relative humidity (10 times less hygroscopic), being classified as slightly hygroscopic according to FB.

It should be understood that the embodiments described above are merely illustrative and that any modifications thereto may occur to a person skilled in the art. Consequently, the present invention should not be considered limited to the embodiments described herein.

## Claims

1. A Crystalline form of phentermine hydrochloride **characterized by** being the monohydrate form.

2. The Crystalline form, according to claim 1, **characterized by** comprising peaks expressed in degrees 2-theta (± 0.2°) at 13.3; 13.7; 14.3; 14.7; 15.1; 16.0; 18.4; 19.5; 20.5; 21.2; 22.2. 22.7; 23.3; 24.4; 24.8; 25.8; 26.4; 27.4; 28.2; 28.6; 29.1; 29.8; 30.5; 31.6; 32.3; 32.9; 34.2; 34.9; 36.0; 36.6; 37.3; 38.2; 39.5; 40.0; 40.4; 43.1; 43.6; 45.1; 46.1; 47.1; 47.6; 48.9; 52.9 and 54.2 in its X-ray diffractogram.

3. The Crystalline form, according to claim 2, **characterized by** comprising specific peaks expressed in degrees 2-theta (±0.2°) at 13.7; 14.7; 15.1; 16.0; 18.4; 22.2; 24.8; 26.4; 28.6 and 29.8.

4. The Crystalline form, according to any one of claims 1 to 3, **characterized by** comprising the characteristic bands 849, 918, 1030, 1074, 1082, 1151, 1173, 1181, 1134, 1380, 1396, 1449, 1454, 1468, 1528, 1533, 1618, 1632, 1650, 2804, 2894, 2935, 2973, 2987, 3066, 3144, 3148 and 3415 cm-1 in its absorption spectrum in the infrared region wherein the band at 3415 cm-1 is from the water molecule.

5. A Process for obtaining the crystalline form of phentermine hydrochloride, as defined in any one of claims 1 to 4, **characterized by** comprising the steps of: (a) High shear granulation of phentermine hydrochloride in anhydrous form; (b) Addition of granulating solution in the mixture of the granulate from (a); (c) Mixture of the granulate of (a) and the granulating solution of (b) obtaining a powder; and, (d) Drying of the powder in a fluidized bed or in an oven or in a vacuum oven.

6. The process, according to claim 5, **characterized by** said granulating solution of step (b) being water added at a speed equal to or above 5 mL/min and in that step (c) has a mixing speed of at least 100 rpm.

7. The process, according to claim 5, **characterized by** in step (d) the powder being dried in a fluidized bed with an inlet air temperature of a maximum of 60 °C and the drying occurs until the pure monohydrated form being obtained.

8. A process for obtaining the crystalline form of phentermine hydrochloride, as defined in any one of claims 1 to 4, **characterized by** comprising the steps of: (a) Recrystallization of the phentermine hydrochloride anhydrous form suspension or solution in water or in a mixture of water with volatile solvents; and (b) Drying in an oven or vacuum oven or vacuum filtration of the solution.

9. The process, according to claim 8, **characterized by** said recrystallization of the solution being at low temperature or with heating and in that said recrystallization of the suspension is maintained under magnetic stirring and at a temperature of 25 °C for 24h.

10. The process, according to claim 9, **characterized by** said low temperature recrystallization taking place between 2 and 8 °C.

11. The process, according to claim 9, **characterized by** said recrystallization with heating being above 60 °C.

12. A process for obtaining the crystalline form of phentermine hydrochloride, as defined in any one of claims 1 to 4, **characterized by** comprising the steps of: (a) Exposure of the anhydrous form of phentermine hydrochloride to an atmosphere with high humidity in a controlled humidity chamber; and (b) Drying in an oven or vacuum oven or vacuum filtration of the solution.

13. The process, according to claim 12, **characterized by** said high humidity condition being between 90 to 95% RH.
